# EUROPEAN PATENT SPECIFICATION

(11) **EP 3 558 407 B1**
(45) Date of publication and mention of the grant of the patent: **15.03.2023**
(21) Application number: 17816872.0
(22) Date of filing: 19.12.2017
(51) Int. Cl.: A61L 31/08, A61L 31/16, A61L 29/08, A61L 29/16

(54) **DRUG RELEASING COATINGS FOR MEDICAL DEVICES AND METHODS OF MAKING SAME**
ARZNEIMITTELABGEBENDE BESCHICHTUNGEN FÜR MEDIZINISCHE VORRICHTUNGEN UND VERFAHREN ZUR HERSTELLUNG DAVON
REVÊTEMENTS LIBÉRANT UN MÉDICAMENT POUR DISPOSITIFS MÉDICAUX ET PROCÉDÉS POUR LES PRODUIRE

(30) Priority: 22.12.2016 US 201662437685 P
(43) Date of publication of application: 30.10.2019
(73) Proprietor: Biotronik AG, 8180 Bülach (CH)
(72) Inventor: BETTS, Ronald E., La Jolla California 92037 (US); NGUYEN, John Dang, San Diego California 92129 (US)
(74) Representative: Biotronik Corporate Services SE
(86) International application number: PCT/EP2017/083614
(87) International publication number: WO 2018/114992

(56) References cited:
- EP-A1- 2 793 967
- WO-A1-2013/182503
- WO-A2-2007/047781
- US-A1- 2006 069 427
- US-A1- 2007 104 753

## Description

### FIELD OF THE INVENTION

This application relates generally to the field of drug releasing insertable devices and methods of making and using same. Specifically, this technology relates to controlled release of drug from a coated balloon directly into affected tissue regions within the body of an individual.

### BACKGROUND OF THE INVENTION

Atherosclerosis is a chronic inflammatory response in the walls of arteries that, over time, has disastrous effects on arterial blood vessels and arterial walls, and can result in formation of fatal plaques as cells accumulated. Coronary artery disease (CAD) is a widespread disease, affecting millions of men and women in the US and is often associated with atherosclerosis. In many patients with CAD, percutaneous transluminal coronary angioplasty (PTCA) is a widely used treatment method, which involves the widening or expansion of the blood vessel walls through mechanical means, generally involving a balloon or other similar implant. The employment of PTCA has been a viable option for the last four decades, though many angioplasty techniques have been limited by frequently occurring complications, including vessel dissection, restenosis, recoil and thrombosis.

During the healing process, inflammation caused by angioplasty and mechanical implant injury often causes smooth muscle cell proliferation and regrowth inside the mechanical implant, thus partially closing the flow channel, and thereby reducing or eliminating the beneficial effect of the angioplasty/implant procedure. Blood clots may also form inside of the newly inserted mechanical implant due to the thrombotic nature of such implant surfaces, even when biocompatible materials are used to form such implant. Restenosis results in significant more morbidity and mortality and frequently necessitates further interventions such as repeated angioplasty or coronary by-pass surgery.

Mechanical implants have historically taken one of two forms: a balloon catheter, capable of inflating and deflating, or a radially expandable, metal stent. Both devices were developed with the idea of maintaining the opening or widening of the blood vessel after an angioplasty procedure. However, both implants have had a history of initial complications, resulting in progressive innovations beyond the initial designs. While the use of balloon angioplasty has revolutionized coronary revascularization through recanalizing narrowed coronary arteries and endovascular vessels, such methods were associated with subintimal dissection, abrupt vessel closure and restenosis (Gruentzig, A., Am Heart J 103:779-783 (1982)). The advent of the implanted stent resolved most issues related to dissection and resolved elastic recoil and late negative vessel remodeling. However there was often found to be increased inflammation in the vessel wall, resulting in an increased risk of intimal hyperplasia, which lead to the potentially fatal in-stent restenosis (Serruys et al., N Engl J Med 331:489-495 (1994)); Hoffman et al., Circulation 94:1247-1254 (1996)).

Eventually, drug eluting stents replaced bare metal stents as a way to reduce the risk associated with restenosis and cellular proliferation at the target site. Unfortunately, even this innovation was wrought with complexities, including elevated risks of very late stent thrombosis (blood clotting within the stent) resulting from localized inflammation, usually leading to prolonged antiplatelet therapy in order to resolve the thrombotic event (Camenzind et al., Circulation 1115:1440-1455 (2007)).

Such limitations with certain drug eluting stents gave rise to a reevaluation of other mechanical implants, including the development of certain non-stent based delivery platforms, such as drug eluting balloons. One of the major studies of its kind (PEPCAD II) examined 131 patients having in-stent restenosis, with the patients being randomly divided into two treatment groups: one group treated by a paclitaxel-coated balloon and the other group treated with a paclitaxel eluting stent. The results of the PEPCAD II study found patients treated with drug eluting balloons experienced a 6-fold decrease in restenosis events and a 5-fold decrease in material adverse coronary events when compared to those patients in the drug eluting stent group (Unverdorben et al., Circulation 119:2986-2994 (2009)).

Studies such as the PEPCAD II have provided suitable evidence for the value of drug eluting balloons when compared to use of drug eluting stents, particularly in scenarios where stents cannot be used, including smaller vessels or calcified/bifurcated lesions, where there is a risk of stent fracture.

There have been a number of publications detailing the innovations in the state of the art of balloon catheters.

U.S. Patent Publication No. 20040224003 describes medical devices such as stents and balloons having an oil based coating suitable for delivering water insoluble therapeutic agents. There is no mention of lipids or albumin with respect to the oil based coatings described therein.

PCT Publication No. WO 97/35575 describes the macrocyclic triene immunosuppressive compound everolimus and related compounds for the treatment of restenosis as a consequence of proliferation and migration of intimal smooth muscle cell as induced by vascular surgeries such as angioplasty.

U.S. Patent Publication No. 20070264303 describes an implantable medical device for delivering a therapeutic agent further comprising an inorganic or ceramic oxide, such as titanium oxide.

U.S. Patent Publication No. 20100285085 describes the use of a coating exhibiting drug transfer control through modulation of coating thickness for application to a balloon catheter. This coating may be applied to a balloon catheter or, optionally, a stent, with less than 30% of the coating to remain on the balloon or stent after delivery to a vessel.

U.S. Patent Publication No. 20140046254 teaches a drug delivery balloon having a coating which includes a therapeutic agent, an excipient and a plasticizer, wherein at least 30% of the coating transfers from the balloon surface within one to two minutes after inflation of the balloon.

U.S. Patent No. 6,171,609 teaches the use of an intravascular stent comprising a coating of taxol in order to inhibit or reduce restenosis following placement of the stent. Also disclosed are methods for the direct and/or targeted delivery of therapeutic agents to vascular smooth muscle cells that cause a dilation and fixation of the vascular lumen by inhibiting smooth muscle cell contraction, thereby constituting a biological stent.

U.S. Patent No. 6,258,121 discloses a stent having a polymeric coating for controlled release of an agent, wherein the polymeric coating a first and second polymeric material, as well as the use of PLA-PEO and PLA-PCL copolymers in order to control the pace of the drug release.

U.S. Patent No. 8,114,049 describes catheter devices having an expandable balloon for delivering a therapeutic agent to a body site. The expandable balloon is capable of being foldable in a variety of configurations and may be coated with a contrast agent or carbohydrate, either separately or blended with the therapeutic agent of interest. The expandable balloon is also folded with one or more packets that contain the therapeutic agent to be delivered.

U.S. Patent No. 8,066,404 describes synthetic biocompatible terpolymer, co-polymer and homopolymer compositions for drug releasing medical devices. The polymers disclosed allow for release of the drug substance from the medical devices.

U.S. Patent 8,414,525 describes balloon catheter devices coated with therapeutic agents with additive materials other than oils or lipids that specifically do not crack upon expansion of the balloon. This technology is specifically designed to avoid cracking upon expansion of the balloon. One embodiment includes albumin as an emulsion in an additive layer coupled with the drug in the same layer in order to avoid the cracking.

EP 2 793 967 A1, published as WO 2013/092416 A1 discloses medical devices with a lipophilic lubricant coating. WO 2007/047781 A1 discloses medical devices having hydrophobic crosslinked gels as coating. US 2006/069427 A1 discloses stents having a substantially polymer-free drug coating. US 2007/104753 A1 discloses stents having a coating with therapeutic agent in it. WO 2013/182503 A1 discloses derivatives of rapamycin.

The current state of the art in existing drug eluting balloon technology comes with several potential advantages, including consistent drug transfer from the balloon to the vessel wall, rapid release of drug, at higher concentrations over a shorter period of time, when compared to drug eluting stents, lack of dependency on antiplatelet therapy (given the localized delivery of the drug into the vessel wall) and overall lower risks of chronic inflammation and late stage thrombosis. In cases of bifurcated or smaller vessels, the absence of a stent allows the original anatomy of the artery to remain intact, thereby reducing the possibility of abnormal flow patterns.

The literature describes various ideas regarding the mechanisms behind the transfer of drug from balloon surface to vessel wall. Proposed theories range from the drug fracturing or dissolving from the coating upon balloon expansion to the drug permeating into the vessel wall via direct contact with the coating on the balloon surface. Regardless of the precise mechanism behind drug transfer from a balloon, a constant of the state of art is the need for rapid delivery of a specific drug concentration during balloon expansion.

There have been drug eluting balloons that have utilized sirolimus or sirolimus derivatives, though preclinical testing have shown these to be relatively ineffective. This may be the fault of the specific coating used or an inability of the drug to provide the desired therapeutic endpoint once the balloon has been expanded at the target site, either because of lower than expected drug concentration or the drug is rapidly metabolized once in the target tissue.

There are several known issues that evidence limitations to modern drug eluting balloons. A major concern is the acute recoil observed in vessels post-balloon angioplasty, and it remains unclear where drug eluting balloons can resolve the late negative remodeling evidenced in studies with non-coated balloons. Furthermore, current investigations reveal complexities in present-day coatings with drugs in order to arrive at the desired release profiles required in certain procedures, where difficulties lie in striking a balance between maintaining the drug during insertion but allowing the drug to be released at the target site once the balloon is inflated.

Enhanced permeability and retention (EPR) effect, originally reported by Matsumura and Madea (Matsumura Y., Cancer Research 46:6387-6392 (1986)), is a phenomenon whereby in contrast to normal tissues and organs, cancerous tissue can take up circulating molecules of certain sizes from systemic blood. Once taken up, these molecules tend to be retained and accumulate in the tissue against the tissue/plasma concentration gradient. It is thought that this is due to unique abnormalities of tumor vasculature. These abnormalities include hypervascularization as well as defective vascular architecture with resulting large gaps between endothelial cells. This and the lack of vascular shielding smooth muscle layers allows dissolved molecules from the intravascular fluid to leak preferentially from tumor blood vessels and accumulate in tumor tissues. These molecules are then retained in the tumor bed due to reduced lymphatic drainage in the tumor interstitium. Intratumor retention also is enhanced by production of various vascular permeability mediators such as Bradykinin, vascular endothelial growth factor (VEGF), nitric oxide, prostaglandins and other permeability enhancing factors (Maeda et al., J. Control. Release 65:271-284 (2000)). It is generally accepted that for optimum EPR, proteins and synthetic polymers require a molecular weight greater than approximately 40 kDa whereas smaller molecules are free to diffuse back out of the tumor tissue. (Maeda et al., Adv.Drug Deliv. Rev., 65:71-79 (2012)). EPR has been demonstrated with various preclinical human tumor animal models and now recently with human clinical studies using systemically administered polymer conjugate of camptothecin (Clark, A.J., PNAS 113 14:3850-3854 (2016)). Tumor and adjacent nonneoplastic tissue biopsies were obtained from patients. Both pre- and postdosing tissue samples adjacent to tumors showed no evidence of conjugated drug. Predose tumor samples showed similar results whereas postdose tumor samples were found to contain active drug.

Importantly, there is new evidence that EPR effects are also present in atherosclerotic tissues. To validate a microfluidic in vitro model, Langer studied nanoparticle translocation in an in vivo rabbit model of atherosclerosis (Kim et al., PNAS 111 3:1078-1083 (2014)). New Zealand White rabbits were fed a high cholesterol diet to induce atherosclerotic lesions. One test cohort consisted of i.v. injection of Gadolinium labeled nanoparticles. A second cohort received i.v. injection of Cy7 labeled albumin, whereas control cohorts three and four consisted of normal diet, healthy animals each receiving the respective test substance. Imaging results revealed that translocation of nanoparticles and albumin occurred primarily in aortic vessels of the cholesterol induced atherosclerotic groups. These groups were shown to have an abundance of permeable neovessels reaching into the atherosclerotic plaque. It was suggested that the Cy7 labeled albumin served as a tracer of vascular permeability. Collectively these results strongly point to defective cellular and tissue architecture shared in both solid cancer tumors and atherosclerotic tissues with functional EPR effects in both. While recognized as important in anti-tumor targeting, EPR has been little exploited in atherosclerotic disease treatment as either a diagnostic or targeted drug delivery means. In addition to the facilitation of drug release of the present invention, EPR effects can also deliver and result in the accumulation of albumin bound drugs within atherosclerotic tissue. Albumin has shown effective binding for a variety of drug agents including paclitaxel nanoparticles (Kratz, F., J. Control. Release 132: 171-183 (2008))

There is a need in the state of the art to provide a drug eluting balloon technology that allows for a safe and effective delivery of a drug into a target tissue, wherein the pharmacokinetics and dosing profile may be customized based on specific circumstances of the individual to be treated. Additionally, there exists a need for a drug eluting balloon capable of implantation at a target site, where the drug remains on the balloon surface during catheter insertion and is only released once the balloon is inflated or deployed at the target site, with the drug being retained at the target site at a therapeutically effective concentration over a period of time.

### SUMMARY OF THE INVENTION

The present invention provides for a drug eluting balloon catheter, comprising a primer drug releasing layer containing a water soluble, preferably neat, material layer and a multi-part drug layer having a first part and a second part, wherein the first part comprises a macrocyclic triene immunosuppressive compound and the second part comprises at least one polymer-free excipient, wherein polymer-free excipient is a fatty alcohol or fatty aldehyde or a fatty acid or a nonionic surfactant.

In one embodiment the polymer-free excipient is selected from fatty alcohols or fatty aldehydes as defined herein and is preferably selected from fatty alcohols. The polymer-free excipient can further be saturated or unsaturated, linear or branched and is preferably saturated and linear. The balloon catheter is comprised of a primer drug releasing layer and a drug layer, wherein the drug layer comprises a macrocyclic triene immunosuppressive compound. Optionally, the drug releasing layer is allowed to dry prior to the application of the drug layer. Preferably, the water soluble material drug releasing layer is globular serum protein and the macrocyclic triene immunosuppressive compound is lipophilic. In a similar aspect, the water soluble material layer is composed of a polymer or a protein having an approximate molecular weight of between 50 to 200 kD. In one embodiment the water soluble material is a polymer or a protein having an approximate molecular weight of between 65-70 kD. In a further embodiment the water soluble material is selected from blood proteins such as globulins and/or fibrinogens having molecular weights up to approximately 160 kD.

In one aspect, the present invention teaches a method of manufacturing a drug eluting balloon catheter comprising: (a) providing a device, preferably a balloon catheter capable of radial expansion once inflated; (b) providing an aqueous solution of a water soluble agent or excipient preferably comprising from about 10% to about 30% of the water soluble agent or excipient in a solution of water; (c) coating, preferably dip coating the device in the solution of (b); (d) allowing the coated device to dry; (e) applying a solution comprising a macrocyclic triene immunosuppressive compound and at least one polymer-free excipient to the device of (d); and (f) allowing the device of (e) to dry.

In one specific aspect, the present invention teaches a method of manufacturing a drug eluting balloon catheter comprising: (a) providing a balloon catheter capable of radial expansion once inflated; (b) providing an aqueous solution of a water soluble agent preferably comprising from about 10% to about 30% of the water soluble agent in a solution of water; (c) dip coating the balloon catheter in the solution of (b); (d) allowing the dip coated balloon catheter to dry; (e) applying a solution comprising a macrocyclic triene immunosuppressive compound and at least one saturated fatty alcohol polymer-free excipient to the balloon catheter of (d); and (f) allowing the balloon catheter of (e) to dry.

In another aspect, the present invention provides a medical device comprising a catheter with a balloon (balloon catheter) having a primer coating layer comprising a water soluble agent and a second, preferably outermost layer containing a fatty alcohol or fatty aldehyde lipid excipient and a drug, wherein the water soluble agent is a globular serum protein, the fatty alcohol or aldehyde lipid excipient is a nonionic, linear hydrocarbon and the drug is a lipophilic, macrocyclic triene immunosuppressive compound, further wherein the outermost coating layer is capable of forming cracks after the balloon catheter is deployed.

### BRIEF DESCRIPTION OF THE DRAWINGS

The disclosed subject matter contained herein is best described in conjunction with the accompanying drawings, in which:

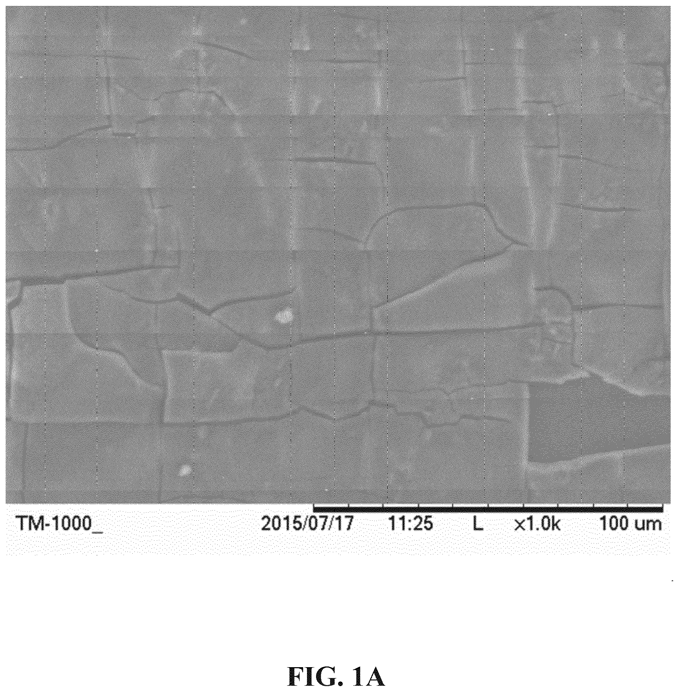

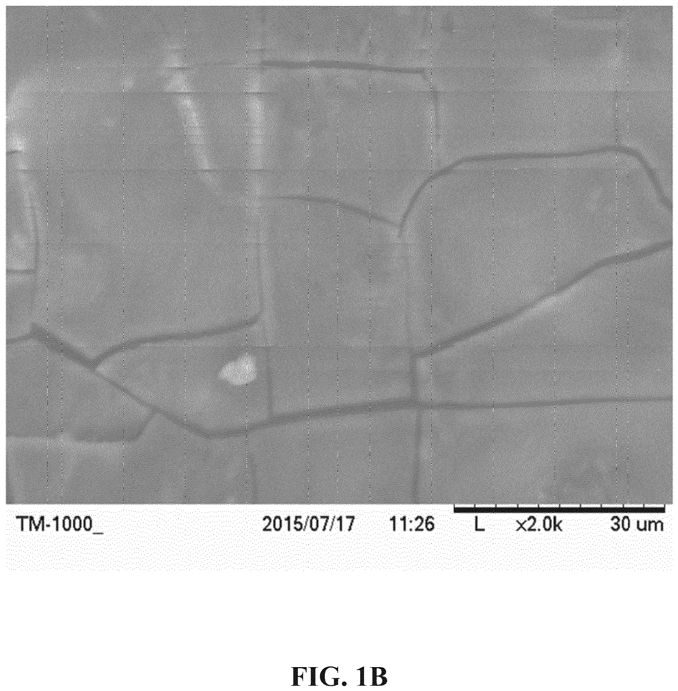

Figure 1 depicts a photomicrograph evidencing micro-cracks within the outermost, lipid-drug coating layer after balloon expansion. (A) at 1000X resolution; (B) at 2000X resolution.
Figure 2 shows two catheters each containing a nylon balloon and coated with a lipophilic blue dye. (A) Catheter A contained a nylon balloon coated with a lipophilic blue dye simulating the drug embodiment of the present invention; (B) Catheter B contained a nylon balloon first coated with human serum albumin followed by the same amount of lipophilic blue dye as in Catheter A.

### DETAILED DESCRIPTION OF THE INVENTION

### Definitions

As used herein, the term "coated" or "coating" with reference to a balloon catheter refers to compounds or substances applied to the balloon surface or otherwise integral to the balloon surface. This would include a drug or therapeutic compound, as well as any other substance that is applied to the balloon surface in order to facilitate the delivery of the drug from the balloon surface to a target.

As used herein, the term "elution" refers to the transfer of a drug or therapeutic agent out of the coating layer and is determined as the total amount of the drug or therapeutic agent excreted out of the coating layer as formulated for application to the surface of the balloon catheter of the present invention.

As used herein, the term "excipient" refers to a natural or synthetically derived substance that is formulated in conjunction with an active agent.

As used herein, the term "lipids" refers to any of a group of organic compounds, including the fats, oils, waxes, sterols, and triglycerides, that are largely insoluble in water but soluble in nonpolar organic solvents, and are oily to the touch.

As used herein, the term "target" or "target tissue" refers to the therapeutic endpoint for the devices of the present invention. Such tissues may include, but are not limited to, blood vessels, interior vasculature areas and regions at the interface between organs and the vasculature of an individual.

As used herein, the term macrocyclic triene immunosuppressive compound includes rapamycin (sirolimus), everolimus, zotarolimus, biolimus, novolimus, myolimus, temsirolimus and the rapamycin derivatives described in this disclosure. In one embodiment the macrocyclic triene immunosuppressive compound is selected from the group comprising or consisting of rapamycin (sirolimus), everolimus, zotarolimus and biolimus. Also, the multi-part drug layer may contain tacrolimus, paclitaxel, docetaxel or analogs thereof or mixtures thereof as drug.

The present invention provides for a balloon catheter, and methods of making and using same, having a unique multi-layer coating formulated for application to the external surface of the balloon to be inserted and deployed at the target site. The embodiments of the present invention are designed for insertion into the body of an individual in order to be applied to a target tissue or site of interest. Such non-limiting examples of the application of the devices of the present invention include for use in the treatment of diseases or conditions of the vasculature system, but also include specific targets (ie. internal organs, certain tissue types or internal regions) that would benefit from the therapeutic uses described herein. Also, the devices to which the coating as suggested herein can be applied are not limited to balloon catheters, but can also other devices which benefit from a fast and efficient drug release.

The present invention provides for a drug eluting balloon catheter having a coating formulated as follows: a primer layer comprising a water soluble material and a multi-part drug layer, wherein the multi-part drug layer further comprises a macrocyclic triene immunosuppressive compound and at least one fatty alcohol or fatty aldehyde, polymer-free excipient.

Preferably, the primer layer of the multi-layer coating is comprised of a water soluble material and forms the first coating layer on the surface of the balloon catheter. This primer layer is preferably applied via dip coating, wherein the balloon catheter is placed into a solution comprising the water soluble material, which is applied to the surface of the balloon catheter. Other suitable methods such as spraying or application of a solution by a thread, a needle, a cannula, a sponge or a piece of cloth can be used for the coating procedure. Following this application of the primer layer, the balloon catheter is removed from the solution or the application device and allowed to dry, for example at ambient room temperature for a time less than 24 hours

The water soluble material that is meant to comprise the bulk of the primer layer is a polymer or a protein having an approximate molecular weight of between 50 to 200 kD. In one embodiment the water soluble material is selected from water soluble human serum proteins or water soluble blood proteins preferably having an approximate molecular weight of between 50 to 200 kD. In one embodiment the water soluble material is a polymer or a protein having an approximate molecular weight of between 65-70 kD, preferably a globular serum protein having an approximate molecular weight of between 65-70 kD. In a further embodiment the water soluble material is selected from blood proteins such as globulins and/or fibrinogens having molecular weights up to approximately 160 kD. More preferably, the water soluble material is human fibrinogen or immunoglobulin. Most preferably, the water soluble material is a human serum protein having at least 90% identity to the following sequence:











 (SEQ ID NO: 1)

In a most preferred embodiment of the invention the water soluble excipient is human serum albumin.

Once the primer layer has dried, a multi-part drug layer is applied to the surface of the balloon catheter and on top of the dried primer layer. The multi-part drug layer is formulated to comprise at least two parts: a first part comprising a drug and a second part comprising a polymer-free excipient as defined herein. In a preferred embodiment, the multi-part drug layer is formulated as a single formulation. The drug of the first part of the multi-part drug layer is a macrocyclic triene immunosuppressive compound. More preferably, the drug is a macrocyclic triene immunosuppressive compound having the following structure: 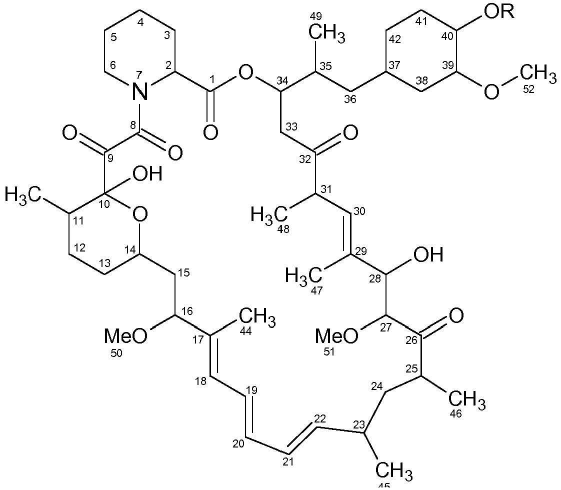 where R is C(O)-(CH₂)ₙ-X, n is 0, 1 or 2, X is a cyclic hydrocarbon having 3-8 carbons and optionally contains one or more unsaturated bonds. In a most preferred embodiment, C(O)-(CH₂)ₙ-X has one of the following structures:

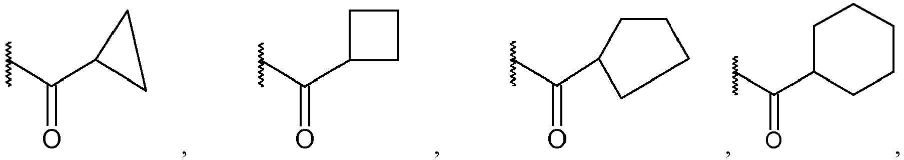

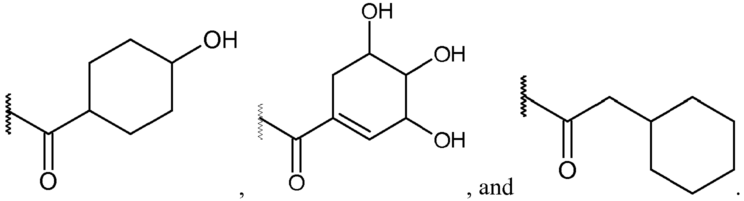

The excipient of the second part of the multi-part drug layer is a non-polymer, non-ionic, linear hydrocarbon or surfactant selected from the group consisting of a lipoic fatty alcohol or a fatty aldehyde or a fatty acid or combinations thereof. Preferably, the excipient is a member selected from the group consisting of lauryl alcohol, undecyl alcohol, myristyl alcohol, pentadecyl alcohol, palmitoleyl alcohol, palmityl alcohol, isocetyl alcohol, heptadecanol, lanolin alcohol, stearyl alcohol, isostearly alcohol, 12-hydroxystearyl alcohol, heneicosyl alcohol, behenyl alcohol, erucyl alcohol, 1-tricosanol, lignoceryl alcohol, 1-pentacosanol, ceryl alcohol, 1-heptacosanol, montanyl alcohol, 1-nonacosanol, myricyl alcohol, 1-hentriacontanol, lacceryl alcohol, 1-tritriacontanol, geddyl alcohol, arachidic acid, behenic acid, lignoceric acid and cerotic acid and the like as well as the aldehyde version of each.

Preferably the fatty alcohol or fatty aldehyde or nonionic surfactant is linear and contains at least 12 carbon atoms. Preferably, the coating comprises a compound having the formula CₓH_{y}O, wherein x is at least 12 and y is at least 22, preferably x is at least 16 and y is at least 28. Yet more preferably, the coating comprises a compound having the formula CₓH_{y}O, wherein x is at least 12 and at the most 34, and y is at least 22 and at the most 70. In one further embodiment, the coating comprises a compound having the formula CₓH_{y}O, wherein x is at least 16 and y is at least 28 and the compound is a non-polymer, linear, branched or cyclic, saturated or unsaturated fatty alcohol or fatty aldehyde. Yet more preferably, the coating comprises a compound having the formula CₓH_{y}O, wherein x is at least 18 and at the most 24, and y is at least 32 and at the most 50, and wherein the compound may further be a non-polymer, linear, branched or cyclic, saturated or unsaturated fatty alcohol or fatty aldehyde, and preferably a linear, saturated fatty alcohol.

In one embodiment, the formulation of the multi-part drug layer comprises at least 80% by weight of the drug as defined herein and at least 15% by weight of at least one polymer-free excipient as defined herein. In a preferred embodiment of the invention, the formulation of the multi-part drug layer comprises from 60 to 95% by weight of the drug as defined herein and 5 to 40% by weight of at least one polymer-free excipient as defined. The formulation may further comprise an adequate amount of a solubilizing agent, such as a suitable organic solvent, and particularly a nonpolar organic solvent to facilitate suitable application of the formulation such as spray coating. Similarly, the amount of the drug as defined herein applied per drug eluting device, and in particular per balloon catheter is between 5 µg to 25 mg, preferably from about 1 mg to about 10 mg, depending on implant size. The amount of the polymer-free excipient is between 1 µg to 16.7 mg, preferably from about 2 µg to about 2.9 mg. In a most preferred embodiment, the drug load of the drug as defined herein per unit length of the catheter balloon from about 0,5 µg/mm² to 10 µg/mm² and preferably from about 1 to 3 µg/mm^{2.}.

In a preferred embodiment, the multi-part drug layer comprising the first part and the second part is applied to the surface of the balloon after the primer layer has dried. In a preferred embodiment the multi-part drug layer is applied via spray coating on top of the primer layer. In one aspect, the multi-part drug layer is vacuum dried at a temperature higher than ambient room temperature, preferably in the range of 30 to 50 °C and most preferably at 40 °C.

### Examples

### Example Formulations:

The macrocyclic triene immunosuppressive compound of the present invention has more than one embodiment and may be described as comprising at least one of the following species from Table 1:

**Table 1**

| Description of CRC-015 species | | |
|---|---|---|
| Main structure | R is C(O)-(CH2)n-X having one of the following structures | Species |
| 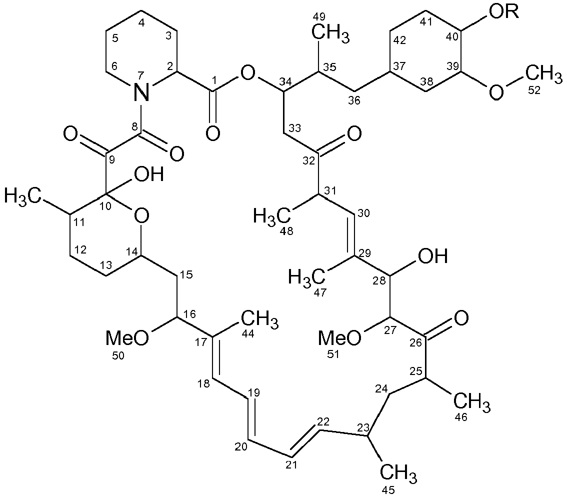 | 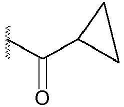 | CRC-015a |
| | 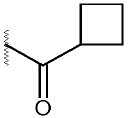 | CRC-015b |
| | 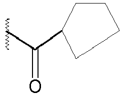 | CRC-015c |
| | 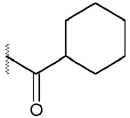 | CRC-015d |
| | 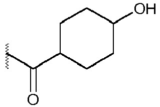 | CRC-015e |
| | 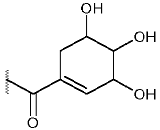 | CRC-015f |
| | 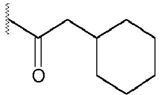 | CRC-015g |

CRC-015 is a term meant to encompass a genus and used to refer to each of the following species from Table 1: CRC-015a, CRC-015b, CRC-015c, CRC-015d, CRC-015e, CRC-015f and CRC-015g.

### I. Formulation and coating of balloon surface with the drug release layer

### Drug releasing layer formulation No.1:

A solution containing 20% of the water soluble, serum protein in water is prepared. The balloon catheter is dipped into the solution and left for a period of time. The surface-bound releasing agent, known as the primer layer, is allowed to dry at ambient temperature for less than 24 hours.

The serum protein of the primer layer is water soluble and serves a dual purpose of being both a balloon releasing agent (for the drug layer coated thereafter) and a tissue targeting agent. The latter occurs once the balloon is deployed at the target site of interest, where the drug is released through diffusion kinetic effects and specific EPR metabolic features associated with serum protein used in the primer layer.

### II. Formulation and coating of the balloon surface with the multi-part drug layer

The multi-part drug layer is formulated to comprise two parts: a first part comprising a drug and a second part comprising a lipid excipient. The drug of the first part of the multi-part drug layer is a macrocyclic triene immunosuppressive compound. The excipient of the second part of the multi-part drug layer is a polymer-free, saturated or unsaturated fatty alcohol or fatty aldehyde.

Specific examples of drug layer formulation (Formulation Nos. 1-20):
1. Weigh 6.25 mg 1-hexadecanol (CAS 36653-82-4, Aldrich) into a 10 mL glass vial and add 5 mL acetone. Cap the vial with a PTFE lined cap. Vortex the solution until solids have dissolved. This is labeled as 1.25 mg/mL STOCK 1-hexadecanol. Weigh 6.25 mg CRC-015 into a 3 mL glass vial and add 1 mL STOCK solution. Cap the vial and vortex the solution until solids dissolve. This formulation can be used for device coating.
2. Weigh 6.25 mg 1-octadecanol (CAS 112-92-5, Aldrich) into a 10 mL glass vial and add 5 mL acetone. Cap the vial with a PTFE lined cap. Vortex the solution until solids have dissolved. This is labeled as 1.25 mg/mL STOCK 1-octadecanol. Weigh 6.25 mg CRC-015 into a 3 mL glass vial and add 1 mL STOCK solution. Cap the vial and vortex the solution until solids dissolve. This formulation can be used for device coating.
3. Weigh 6.25 mg 1-hexadecanol into a 10 mL glass vial and add 5 mL chloroform. Cap the vial with a PTFE lined cap. Vortex the solution until solids have dissolved. This is labeled as 1.25 mg/mL STOCK 1-hexadecanol. Weigh 6.25 mg CRC-015 into a 3 mL glass vial and add 1 mL STOCK solution. Cap the vial and vortex the solution until solids dissolve. This formulation can be used for device coating.
4. Weigh 6.25 mg 1-hexadecanol into a 10 mL glass vial and add 5 mL tert-butyl methyl ether. Cap the vial with a PTFE lined cap. Vortex the solution until solids have dissolved. This is labeled as 1.25 mg/mL STOCK 1-hexadecanol. Weigh 6.25 mg CRC-015 into a 3 mL glass vial and add 1 mL STOCK solution. Cap the vial and vortex the solution until solids dissolve. This formulation can be used for device coating.
5. Weigh 6.25 mg 1-hexadecanol into a 10 mL glass vial and add 5 mL 1-chlorobutane. Cap the vial with a PTFE lined cap. Vortex the solution until solids have dissolved. This is labeled as 1.25 mg/mL STOCK 1-hexadecanol. Weigh 6.25 mg CRC-015 into a 3 mL glass vial and add 1 mL STOCK solution. Cap the vial and vortex the solution until solids dissolve. This formulation can be used for device coating.
6. Weigh 6.25 mg 1-docosanol (CAS 30303-65-2 Aldrich) into a 10 mL glass vial and add 5 mL chloroform or 5 mL methyl tert-butyl ether. Cap the vial with a PTFE lined cap. Vortex the solution until solids have dissolved. This is labeled as 1.25 mg/mL STOCK 1-docosanol. Weigh 6.25 mg CRC-015 into a 3 mL glass vial and add 1 mL STOCK solution. Cap the vial and vortex the solution until solids dissolve. This formulation can be used for device coating.
7. Weigh 6.25 mg 1-docosanal (CAS 57402-36-5, Matrix Scientific) into a 10 mL glass vial and add 5 mL chloroform. Cap the vial with a PTFE lined cap. Vortex the solution until solids have dissolved. This is labeled as 1.25 mg/mL STOCK 1-docosanol. Weigh 6.25 mg CRC-015 into a 3 mL glass vial and add 1 mL STOCK solution. Cap the vial and vortex the solution until solids dissolve. This formulation can be used for device coating.
8. Weigh 6.25 mg 1-tetradecanol (CAS 112-72-1, Aldrich) into a 10 mL glass vial and add 5 mL acetone. Cap the vial with a PTFE lined cap. Cap the vial and vortex the solution until solids have dissolved. This is labeled as 1.25 mg/mL STOCK 1-tetradecanol. Weigh 6.25 mg CRC-015 into a 3 mL glass vial and add 1 mL STOCK solution. Cap the vial and vortex the solution until solids dissolve. This formulation can be used for device coating.
9. Weigh 6.25 mg 1-eicosanol (CAS 57402-36-5, TCI) into a 10 mL glass vial and add 5 mL chloroform. Cap the vial with a PTFE lined cap. Vortex the solution until solids have dissolved. This is labeled as 1.25 mg/mL STOCK 1-eicosanol. Weigh 6.25 mg CRC-015 into a 3 mL glass vial and add 1 mL STOCK solution. Cap the vial and vortex the solution until solids dissolve. This formulation can be used for device coating.
10. Weigh 6.25 mg 1-hexadecanol into a 10 mL glass vial and add 5 mL acetone. Cap the vial with a PTFE lined cap. Vortex the solution until solids have dissolved. This is labeled as 1.25 mg/mL STOCK 1-hexadecanol. Weigh 6.25 mg everolimus into a 3 mL glass vial and add 1 mL STOCK solution. Cap the vial and vortex the solution until solids dissolve. This formulation can be used for device coating.
11. Weigh 6.25 mg 1-hexadecanol into a 10 mL glass vial and add 5 mL acetone. Cap the vial with a PTFE lined cap. Vortex the solution until solids have dissolved. This is labeled as 1.25 mg/mL STOCK 1-hexadecanol. Weigh 6.25 mg sirolimus into a 3 mL glass vial and add 1 mL STOCK solution. Cap the vial and vortex the solution until solids dissolve. This formulation can be used for device coating.
12. Weigh 6.25 mg 1-hexadecanol into a 10 mL glass vial and add 5 mL acetone. Cap the vial with a PTFE lined cap. Vortex the solution until solids have dissolved. This is labeled as 1.25 mg/mL STOCK 1-hexadecanol. Weigh 6.25 mg biolimus into a 3 mL glass vial and add 1 mL STOCK solution. Cap the vial and vortex the solution until solids dissolve. This formulation can be used for device coating.
13. Weigh 6.25 mg 1-docosanol into a 10 mL glass vial and add 5 mL acetone. Cap the vial with a PTFE lined cap. Vortex the solution until solids have dissolved. This is labeled as 1.25 mg/mL STOCK 1-docosanol. Weigh 6.25 mg sirolimus into a 3 mL glass vial and add 1 mL STOCK solution. Cap the vial and vortex the solution until solids dissolve. This formulation can be used for device coating.
14. Weigh 6.25 mg 1-tetradecanol into a 10 mL glass vial and add 5 mL acetone. Cap the vial with a PTFE lined cap. Vortex the solution until solids have dissolved. This is labeled as 1.25 mg/mL STOCK 1-tetradecanol. Weigh 6.25 mg zotarolimus into a 3 mL glass vial and add 1 mL STOCK solution. Cap the vial and vortex the solution until solids dissolve. This formulation can be used for device coating.
15. Weigh 6.25 mg 1-hexadecanol into a 10 mL glass vial and add 5 mL acetone. Cap the vial with a PTFE lined cap. Vortex the solution until solids have dissolved. This is labeled as 1.25 mg/mL STOCK 1-hexadecanol. Weigh 6.25 mg paclitaxel into a 3 mL glass vial and add 1 mL STOCK solution. Cap the vial and vortex the solution until solids dissolve. This formulation can be used for device coating.
16. Weigh 12.50 mg 1-octadecanol into a 10 mL glass vial and add 5 mL acetone. Cap the vial with a PTFE lined cap. Vortex the solution until solids have dissolved. This is labeled as 2.5 mg/mL STOCK 1-octadecanol. Weigh 6.25 mg CRC-015 into a 3 mL glass vial and add 1 mL STOCK solution. Cap the vial and vortex the solution until solids dissolve. This formulation can be used for device coating.
17. Weigh 12.50 mg pentadecanol into a 10 mL glass vial and add 5 mL chloroform. Cap the vial with a PTFE lined cap. Vortex the solution until solids have dissolved. This is labeled as 2.5 mg/mL STOCK pentadecanol. Weigh 6.25 mg CRC-015 into a 3 mL glass vial and add 1 mL STOCK solution. Cap the vial and vortex the solution until solids dissolve. This formulation can be used for device coating.
18. Weigh 6.25 mg 1-triacontanol (CAS 593-50-0, Aldrich) into a 10 mL glass vial and add 5 mL chloroform. Cap the vial with a PTFE lined cap. Vortex the solution until solids have dissolved. This is labeled as 1.25 mg/mL STOCK 1-triacontanol. Weigh 6.25 mg docetaxel into a 3 mL glass vial and add 1 mL STOCK solution. Cap the vial and vortex the solution until solids dissolve. This formulation can be used for device coating.
19. Weigh 6.25 mg 12-hydroxystearyl alcohol (CAS 2726-73-0, Aldrich) into a 10 mL glass vial and add 5 mL tert-butyl methyl ether. Cap the vial with a PTFE lined cap. Vortex the solution until solids have dissolved. This is labeled as 1.25 mg/mL STOCK 12-hydroxystearyl. Weigh 6.25 mg sirolimus into a 3 mL glass vial and add 1 mL STOCK solution. Cap the vial and vortex the solution until solids dissolve. This formulation can be used for device coating.
20. Weigh 6.25 mg oleyl alcohol (CAS 143-28-2, Aldrich) into a 10 mL glass vial and add 5 mL chloroform. Cap the vial with a PTFE lined cap. Vortex the solution until solids have dissolved. This is labeled as 1.25 mg/mL STOCK oleyl alcohol. Weigh 6.25 mg paclitaxel into a 3 mL glass vial and add 1 mL STOCK solution. Cap the vial and vortex the solution until solids dissolve. This formulation can be used for device coating.

The specific macrocylic triene immunosuppressive compound, known herein as CRC015, was formulated with the polymer-free excipient and directly applied, via spray coating, onto the surface of the balloon catheter having the primer layer already applied. A solubilizing agent was used in order to make the saturated fatty alcohol readily soluble and to accommodate the formulation with CRC015 prior to spray coating.

The balloon catheter, now having the multi-part drug layer spray coated on top of the primer layer, is dried at 40° in vacuum.

### III. Drug delivery and transfer mechanism of CRC015-coated balloon for assessing in vivo tissue uptake

During preclinical testing, it was found that CRC015 will bind at high levels with the serum protein of the primer layer (SEQ ID NO: 1) in vivo. It was also discovered that while the serum protein of the primer layer is highly water soluble, CRC015 is highly lipophilic. Accordingly, studies were performed that evidenced the protective properties of CRC015 on the degradative effects on the primer layer during device delivery.

Once the balloon catheter has reached the target tissue, it is deployed or inflated, causing the balloon surface to come in physical contact with the target tissue. After the balloon is deployed, the multi-part drug layer cracks, allowing the infiltration of water molecules in the plasma to interact with the primer layer, which then disassociates from the balloon surface, carrying the bound drug directly into the target tissue, where it continues to elute at a therapeutic rate over extended time.

In vivo drug uptake and retention of drug using the present invention is demonstrated using female hypercholestermic White New Zealand rabbits. To induce atherosclerotic lesions the rabbits were fed a 1% cholesterol plus 6% peanut oil diet (BioServ, Fleming, NJ product F4366). The efficacy of this type of diet on the induction of rabbit hypercholestermia and utility of this model for comparison to human pathogenesis is well validated (Schwartz et al., J. Am. Coll. Cardiol. 44(7): 1373-1385 (2004)). PTCA balloon catheters (3.0 mm in diameter and 22.9 mm in length) were inflated at 1013.25-3039.75 hPa (1-3 atm) and dip coated with drug releasing formulation No. 1 containing human serum albumin (Sigma A9731) and allowed to dry at ambient temperatures for 24 hours. The albumin coated balloon was then spray coated with drug layer formulation No. 6 and then pleated, folded and heat set at 50 degrees C for 5 minutes. The folded balloon was then additionally spray coated with formulation No. 6 to achieve a drug dose of 5.75 µg/mm². The drug coated balloon was vacuum dried at 40 degrees C for 72 hours before use. The endothelial denudation of the rabbits was conducted by a surgical cutdown on the carotid artery and a 4F hemostatic sheath advanced around the aortic arch. A guide wire was advanced to the femoral artery and a 3.0 × 10 mm balloon catheter advanced to the distal iliac artery and inflated. The catheter was pulled proximally to the aortoiliac bifurcation and then deflated. This technique was repeated two additional times. This same technique was also conducted to the contralateral iliac artery. The recovered animals were then fed the custom rabbit diet for 42 days. At the end of this time period the prepared drug coated balloons were advanced in each of the treated iliac arteries and inflated to nominal pressure for 60 seconds before removal. The vessels were harvested after 8 days for drug measurement by LCMS after extraction of tissue at balloon treatment site using acetonitrile. Results are presented below at Table 2.

**Table 2**

| Assessment of In Vivo Uptake of CRC-015 | | |
|---|---|---|
| Animal | Artery | Drug remaining in vessel after 8 days |
| 1 | Right Iliac | 428 ng/mg tissue |
| 2 | Left Iliac | 1326 ng/mg tissue |

Tissue level concentrations after 8 days are significant and demonstrate utility of this invention.

Further comparative studies were performed based on the above in vivo uptake assessment (Study No. 1). Specifically, another study was conducted (Study No. 2) based on Study No. 1, except that normal White New Zealand rabbits were used and the drug dose was reduced to 2.79 µg/mm². Additionally, a final study (Study No. 3) was performed similar to Study No. 1, except normal White New Zealand rabbits were used and drug layer Formulation No. 1 was utilized at a dosing of 2.89 µg/mm². The results of the comparative study are presented below at Table 3.

**Table 3**

| Comparative In Vivo Uptake Studies | | | | |
|---|---|---|---|---|
| **Study No.** | **Vessel Type** | **Number of Days** | **Number of Vessels** | **Normalized Uptake¹ (ng/mg/µg)** |
| 1 | Rabbit Iliac Arteries | 8 | 2 | 0.73 |
| 2 | Rabbit Iliac Arteries | 8 | 5 | 0.66 |
| 3 | Rabbit Iliac Arteries | 8 | 4 | 0.79 |
| MagicTouch Sirolimus² | Rabbit Iliac Arteries | 8 | NA | 0.09 |
| US 2014/0046254 Everolimus | Porcine Coronary Arteries | 3 | NA | 0.02 |
| US 2014/0046254 Zotarolimus | Porcine Coronary Arteries | 7 | NA | 0.04 |

| | | | | |
|---|---|---|---|---|
| ¹: Normalized Uptake as a function of ng drug / mg tissue / µg drug on balloon ²: Concept Medical, Cardiovascular Innovation Pipeline @ EuroPCR, 26 May 2010. NA: Data not available | | | | |

As is evidenced in Table 3, the uptake of drug into the target tissues by the devices of the present invention is increased nearly 8-fold when compared to the devices of the current state of the art.

### IV. Albumin binding of sirolimus (rapamycin)

Sirolimus-albumin binding has previously been reported. (Rapamune Product Insert) To determine if CRC-015 is bound to albumin and to compare CRC-015 to sirolimus binding characteristics, 1.485 mL samples containing 1.4 mg/mL bovine serum albumin (BSA) in normal saline were spiked with 15 uL of either CRC-015 or sirolimus dissolved in acetonitrile to yield final drug concentrations of 10 uM. The drug spiked BSA solutions were mixed by inversion and allowed to equilibrate at ambient temperature for 30 minutes to allow drug binding to the albumin protein. 826 mg ammonium sulfate was next added to each sample and mixed by gentle inversion until the salt had completely dissolved and precipitate formed. Samples were then vortexed for 20 seconds then centrifuged for 6.5 minutes at 7200 × g. To both supernatant and separated precipitate 0.750 mL methanol and 0.250 mL acetonitrile containing internal standard was added to each sample. Samples were tested for drug content by LCMS where the supernatant amount represents unbound drug and the precipitate amount the bound drug. Recovery verifiction tests were performed as above but using solutions of saline plus drug without BSA.

A summary of the percent unbound and bound of CRC-015 or sirolimus are listed below at Table 4. Values reported are the average of a separate test on each of four separate days.

**Table 4**

| Binding Comparison between CRC-015 and Sirolimus | | |
|---|---|---|
| Drug | % Unbound | % Bound |
| CRC-015 | 3.0% | 97.0% |
| Sirolimus | 6.2% | 93.8% |

CRC-015 was determined to have higher binding to albumin. High sirolimus-albumin binding is consistent with previous referenced results.

### V. Comparison of Coating Techniques

The coating methods of the present invention, along with the specialized compounds related thereto, are distinguishable over the present state of the art in drug coated medical devices, namely, catheters. A major technical advantage of this new approach, relative to the current state of the art, is the requirement that the therapeutic material is retained on the balloon surface during placement of the catheter, while simultaneously releasing the therapeutic material at the targeted site.

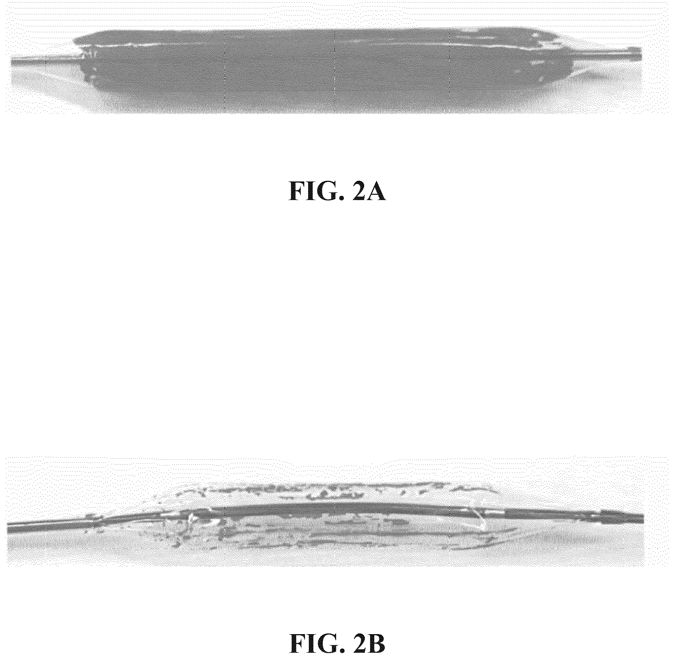

Figure 2 exemplifies the difference between the present invention and the current state of the art with respect to catheters and drug coated balloons. Catheter A contains a nylon balloon coated with a lipophilic blue dye, designed to mimic the CRC-015 compound (FIG. 2A). Catheter B was a similar balloon to that used in Catheter A, but was first coated with a releasing layer comprised of human serum albumin, followed by the same amount of blue dye used on Catheter A (FIG. 2B). Both balloons were expanded while suspended in a static 0.9% NaCl solution. Catheter B was removed from the solution after 30 minutes while Catheter A was allowed to soak for 24 hours. Catheter B releasing layer proved very effective in causing prompt release of the hydrophobic material, as evidenced in FIG. 2B.

## Claims

1. A drug eluting device, comprising a primer layer containing a water soluble material layer and a multi-part drug layer having a first part and a second part, wherein the first part comprises a macrocyclic triene immunosuppressive compound and the second part comprises at least one polymer-free excipient, wherein polymer-free excipient is a fatty alcohol or fatty aldehyde or a fatty acid or a nonionic surfactant, wherein the device is a drug eluting balloon catheter.

2. The device according to claim 1, wherein the water soluble material is a globular serum protein having a molecular weight of between 65-70 kD.

3. The device of one of claims 1 or 2, wherein the water soluble material is human serum albumin.

4. The device of one of claims 1 to 3, wherein the macrocyclic triene immunosuppressive compound has the following structure: 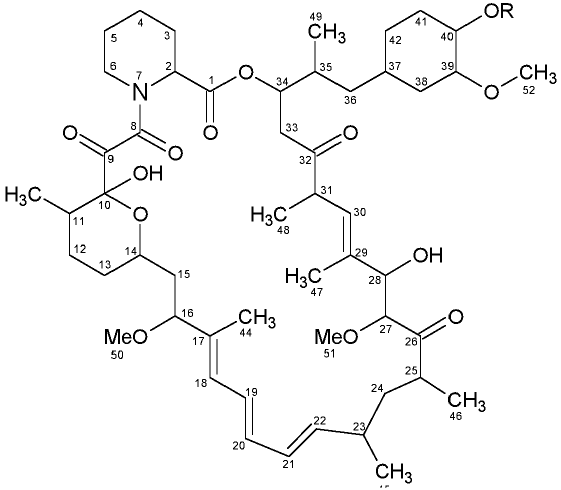 where R is C(O)-(CH₂)ₙ-X, n is 0, 1 or 2, X is a cyclic hydrocarbon having 3-8 carbons and optionally contains one or more unsaturated bonds.

5. The device of claim 4, wherein C(O)-(CH₂)ₙ-X has one of the following structures:

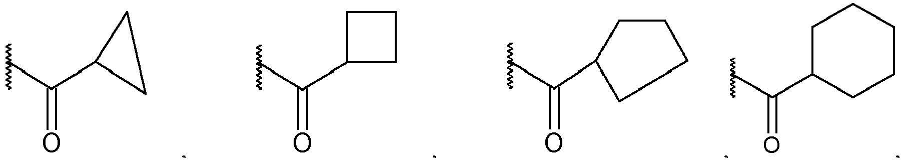

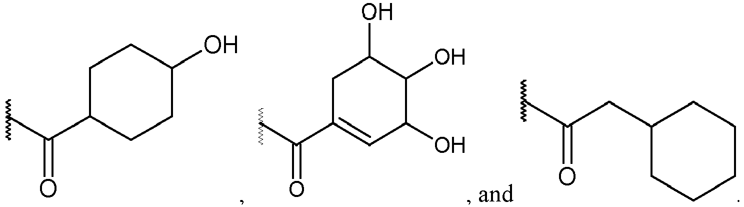

6. The device of one of claims 1 to 5, wherein the macrocyclic triene compound is rapamycin, everolimus, zotarolimus, Biolimus or temsirolimus.

7. The device of one of claims 1 to 6, wherein polymer-free excipient is a linear fatty alcohol or fatty aldehyde or a fatty acid or a nonionic surfactant containing at least 12 carbon atoms.

8. A method of manufacturing a drug eluting balloon catheter, according to one of the claims 1 till 7 comprising: (a) providing a balloon catheter, preferably a balloon catheter capable of radial expansion once inflated; (b) providing an aqueous solution of a water soluble excipient preferably comprising from about 10% to about 30% of the water soluble excipient in a solution of water; (c) dip coating the device in the solution of (b); (d) allowing the dip coated device to dry; (e) applying a solution comprising a macrocyclic triene immunosuppressive compound and at least one saturated fatty alcohol, polymer-free excipient to the device of (d); and (f) allowing the device of (e) to dry.

## Patentansprüche

1. Arzneistofffreisetzende Vorrichtung, umfassend: eine Primerschicht, die eine Schicht aus wasserlöslichem Material und eine mehrteilige Arzneistoffschicht mit einem ersten Teil und einem zweiten Teil enthält, wobei der erste Teil eine immunsuppressive makrocyclische Trienverbindung umfasst und der zweite Teil mindestens einen polymerfreien Hilfsstoff umfasst, wobei der polymerfreie Hilfsstoff ein Fettalkohol oder ein Fettaldehyd oder eine Fettsäure oder ein nichtionisches Tensid ist, wobei die Vorrichtung ein arzneistofffreisetzender Ballonkatheter ist.

2. Vorrichtung nach Anspruch 1, wobei das wasserlösliche Material ein globuläres Serumprotein mit einem Molekulargewicht zwischen 65 und 70 kD ist.

3. Vorrichtung nach einem der Ansprüche 1 oder 2, wobei das wasserlösliche Material humanes Serumalbumin ist.

4. Vorrichtung nach einem der Ansprüche 1 bis 3, wobei die immunsuppressive makrocyclische Trienverbindung die folgende Struktur aufweist: 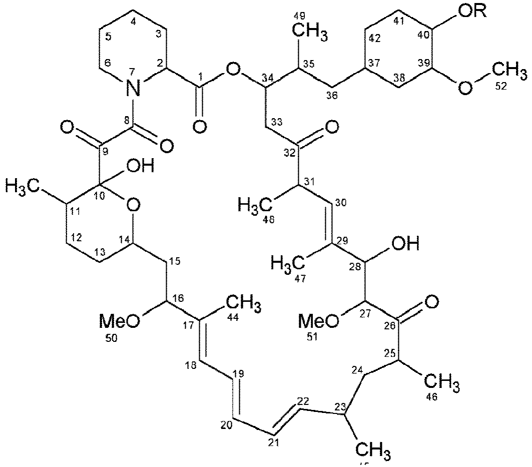 wobei R für C(O)-(CH₂)ₙ-X steht, n 0, 1 oder 2 ist, X ein cyclischer Kohlenwasserstoff mit 3-8 Kohlenstoffen ist und optional eine oder mehrere ungesättigte Bindungen enthält.

5. Vorrichtung nach Anspruch 4, wobei C(O)-(CH₂)ₙ-X eine der folgenden Strukturen aufweist:

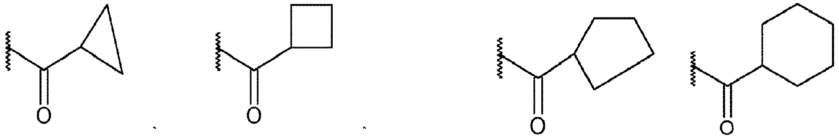

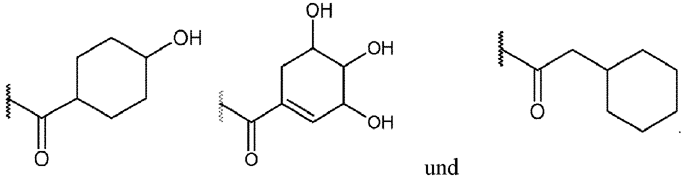

6. Vorrichtung nach einem der Ansprüche 1 bis 5, wobei die makrocyclische Trienverbindung Rapamycin, Everolimus, Zotarolimus, Biolimus oder Temsirolimus ist.

7. Vorrichtung nach einem der Ansprüche 1 bis 6, wobei der polymerfreie Hilfsstoff ein linearer Fettalkohol oder Fettaldehyd oder eine lineare Fettsäure oder ein lineares nichtionisches Tensid mit mindestens 12 Kohlenstoffatomen ist.

8. Verfahren zum Herstellen eines arzneistofffreisetzenden Ballonkatheters nach einem der Ansprüche 1 bis 7, umfassend: (a) Bereitstellen eines Ballonkatheters, vorzugsweise eines Ballonkatheters, der in der Lage ist, sich bei Ausweitung radial auszudehnen; (b) Bereitstellen einer wässrigen Lösung aus einem wasserlöslichen Hilfsstoff, die vorzugsweise etwa 10 % bis etwa 30 % des wasserlöslichen Hilfsstoffs in einer Wasserlösung umfasst; (c) Tauchbeschichten der Vorrichtung in der Lösung von (b); (d) Trocknen lassen der tauchbeschichteten Vorrichtung; (e) Auftragen einer Lösung, die eine immunsuppressive makrocyclische Trienverbindung und mindestens einen polymerfreien Hilfsstoff aus gesättigtem Fettalkohol umfasst, auf die Vorrichtung von (d); und (f) Trocknen lassen der Vorrichtung von (e).

## Revendications

1. Dispositif d'élution d'un médicament comprenant une couche primaire contenant une couche de matériau soluble dans l'eau et une couche de médicament en plusieurs parties ayant une première partie et une deuxième partie, dans lequel la première partie comprend un composé immunosuppresseur triène macrocyclique et la deuxième partie comprend au moins un excipient exempt de polymère, dans lequel l'excipient exempt de polymère est un alcool gras, ou un aldéhyde gras, ou un acide gras, ou un tensioactif non ionique, le dispositif étant un cathéter à ballonnet éluant un médicament.

2. Dispositif selon la revendication 1, dans lequel le matériau soluble dans l'eau est une protéine de sérum globulaire ayant un poids moléculaire entre 65 et 70 kD.

3. Dispositif selon la revendication 1 ou la revendication 2, dans lequel le matériau soluble dans l'eau est de l'albumine de sérum humain.

4. Dispositif selon l'une des revendications 1 à 3, dans lequel le composé immunosuppresseur triène macrocyclique possède la structure suivante : 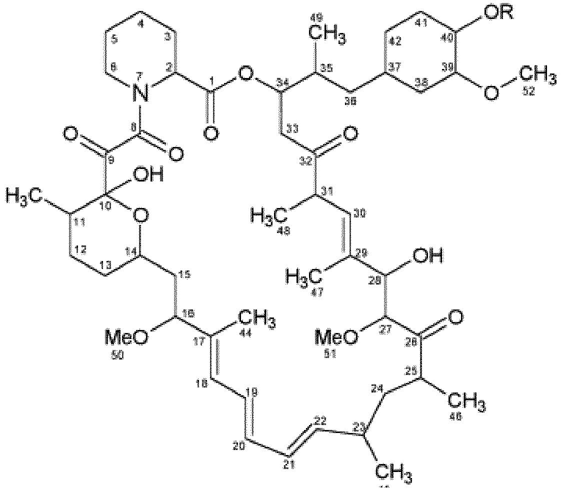 où R équivaut à C(O)-(CH₂)ₙ-X, n est égal à 0, 1 ou 2, X est un hydrocarbure cyclique ayant 3 à 8 atomes de carbone et contenant éventuellement une ou plusieurs liaisons insaturées.

5. Dispositif selon la revendication 4, dans lequel C(O)-(CH₂)ₙ-X possède l'une des structures suivantes :

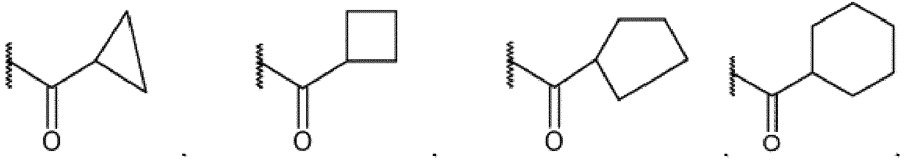

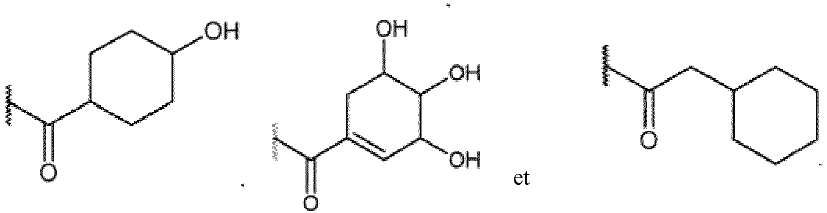

6. Dispositif selon l'une des revendications 1 à 5, dans lequel le composé triène macrocyclique est la rapamycine, l'évorolimus, le zotarolimus, le biolimus ou le temsirolimus.

7. Dispositif selon l'une des revendications 1 à 6, dans lequel l'excipient exempt de polymère est un alcool gras, ou un aldéhyde gras, ou un acide gras, ou un tensioactif non ionique linéaire, contenant au moins 12 atomes de carbone.

8. Procédé de fabrication d'un cathéter à ballonnet éluant un médicament selon l'une des revendications 1 à 7 comprenant : (a) la fourniture d'un cathéter à ballonnet, de préférence un cathéter à ballonnet capable d'être expansé radialement une fois gonflé ; (b) l'alimentation par une solution aqueuse d'un excipient soluble dans l'eau comprenant de préférence environ 10 % à environ 30 % d'excipient soluble dans l'eau dans une solution d'eau; (c) le revêtement par trempage du dispositif dans la solution de (b) ; (d) le fait de laisser sécher le dispositif revêtu par trempage ; (e) l'application d'une solution comprenant un composé immunosuppresseur triène macrocyclique et au moins un alcool gras saturé, un excipient exempt de polymère au dispositif de (d) ; et (f) le fait de laisser sécher le dispositif de (e).
